# EUROPEAN PATENT SPECIFICATION

(11) **EP 1 196 137 B1**
(45) Date of publication and mention of the grant of the patent: **22.12.2004**
(21) Application number: 00945085.9
(22) Date of filing: 30.06.2000
(51) Int. Cl.: A61K 7/16

(54) **SYSTEMS COMPRISING ORGANOSILOXANE RESINS FOR DELIVERING ORAL CARE SUBSTANCES AND FOR PROLONGING SUCH DELIVERY**
ZUSAMMENSTZUNGEN ENTHALTEND ORGANOSILOXAN-HARZE ZUR FREISETZUNG VON MUNDPFLEGEWIRKSTOFFEN UND ZUR VERLÄNGERUNG DER FREISETZUNG
SYSTEME COMPRENANT DES RESINES D'ORGANOSILOXANES PERMETTANT D'ADMINISTRER DES SUBSTANCES DESTINEES A L'HYGIENE BUCCALE ET DE PROLONGER CETTE ADMINISTRATION

(30) Priority: 02.07.1999 WO PCT/US99/15131; 02.07.1999 WO PCT/US99/15130; 09.06.2000 WO PCT/US00/15891; 09.06.2000 WO PCT/US00/15890
(43) Date of publication of application: 17.04.2002
(73) Proprietor: THE PROCTER & GAMBLE COMPANY, Cincinnati, Ohio 45202 (US)
(72) Inventor: YUE, Jiang, West Chester, OH 45069 (US); MITRA, Sekhar, 1 Sha Mian Guangzhou 510133 (CN)
(74) Representative: Clemo, Nicholas Graham
(86) International application number: PCT/US2000/018189
(87) International publication number: WO 2001/001942

(56) References cited:
- US-A- 5 427 770
- US-A- 5 651 959
- US-A- 5 866 630

## Description

### FIELD

The present invention relates to systems for delivering oral care substances to the surfaces of the teeth and for prolonging such delivery. More specifically, the present invention relates to a system comprising a delivery composition comprising organosiloxane resins and at least one oral care substance for delivering the oral care substance, and a protective composition comprised of organosiloxane resins for prolonging the presence of the delivery composition in the oral cavity. The delivery composition forms a film on the surface to which it has been applied and provides sustained release of the oral care substance from the film for prolonged therapeutic, prophylactic, and/or cosmetic benefits. In addition, it is believed that the systems herein may further provide sustained release benefits to other oral surfaces, such as the gingival and mucosal tissues, as well as to the surfaces of the teeth.

### BACKGROUND

Oral care products by which various oral care substances or actives can be delivered to the soft and hard tissues of the oral cavity have previously been known. Examples of such oral care products include, for example, brushing aids such as dentifrice products for delivery of anti-caries actives such as fluoride or other actives for the reduction of the bacteria that lead to the formation of plaque, and mouthwashes containing breath freshening actives and/or antibacterial actives. In addition, bleaching agents such as peroxide that can be applied directly to the surfaces of the teeth, i.e., to the tooth enamel, have been developed.

However, it has been found that such conventional product forms typically do not provide substantivity sufficient to maintain actives on the hard and soft oral tissues for a period of time sufficient to enhance or prolong the therapeutic, prophylactic, and/or cosmetic benefits provided by the actives. Neither have such conventional product forms been able to provide sustained delivery of oral care actives, without periodic reapplication at relatively short time intervals, or without a special delivery device or containment means such as a mouthpiece.

Attempts have previously been made to enhance the substantivity of whitening bleaches, bactericides, and other active components of oral care products. See, e.g., US patent no. 5,425,953 to Sintov et al. on June 20, 1995, in which a film forming, water-soluble cellulosic polymer is used to deliver a bleaching agent to the teeth; US patent no. 5,438,076 to Friedman et al., in which liquid methacrylate acid copolymer compositions are used to deliver a bacteriocidal pharmacological agent; and international Patent Appln. No. WO 97/25968 to Huang, published on July 24, 1997, disclosing a film-coating composition comprising cellulose and polyvinyl acetal, coumarone-indene resin, or shellac as a film former to deliver bleaches to the tooth enamel.

However, the above systems are water-soluble, i.e., they are readily dissolved by saliva, generally within about 1-3 hours after application. Therefore, their degree of durability is low, and they cannot provide long-term delivery of the active ingredient that is present in the composition. In addition, their water-soluble nature precludes them from being used with oral care actives that would be unstable in water-based films. Sodium percarbonate is one example of such an active; it would be unstable in the high pH environment of an aqueous-based film.

In order to provide an applied composition with a relatively higher degree of durability, the use of protective coatings that are applied to the teeth has been described. See, US patent no. 5,401,528, to Schmidt on March 18, 1995, in which organically modified silicic acid polycondensates are deposited on the teeth, then polymerized in-situ by curing, to coat the teeth in order to protect them from plaque deposits. This system is not a true delivery system by which an active ingredient is released over time; instead, it provides a barrier by which the deleterious effect of plaque-causing bacteria may be diminished.

Although such a barrier coating may offer a benefit in terms of enhanced durability, it requires the use of special equipment and complex application; thus, it cannot be performed at home and cannot be used for self-treatment.

US 5,427,770 discloses dentifrice compositions comprising aminoalkylsilicone polymers which deposit from the compositions onto teeth thereby forming a protective barrier. Such deposited barriers to not provide the substantivity of the dry-down films of the present invention.

Therefore, it can be seen that none of these previous developments can offer the combination of both long-term delivery of an oral care substance or active ingredient and the convenience of discrete self-treatment and home use. Based on the foregoing, there is a need for a convenient delivery system for various oral care substances in which the substantivity of the active ingredients is enhanced, and in which the presence of the oral care substance in the oral cavity is prolonged to further enhance the therapeutic, prophylactic, and/or cosmetic benefits provided by the actives. None of the existing art provides all of the advantages and benefits of the present invention.

### SUMMARY

The present invention is directed to systems for delivering an oral care substance to the oral cavity comprising: (a) a delivery composition comprised of: (i) an organosiloxane resin; (ii) a volatile carrier capable of solubilizing the organosiloxane resin; (iii) a rheology modifier; and (iv) at least one oral care substance; and (b) a protective composition comprised of: (i) an organosiloxane resin; and (ii) a volatile carrier capable of solubilizing the organosiloxane resin. The protective composition may further comprise a fluid diorganopolysiloxane-based polymer and/or a rheology modifier.

Preferred systems further comprise a fluid diorganopolysiloxane-based polymer and/or a rheology modifier. In the delivery composition and preferably also in the protective composition.

These and other features, aspects, and advantages of the invention will become evident to those skilled in the art from a reading of the present disclosure.

### DETAILED DESCRIPTION

While the specification concludes with daims particularly pointing out and distinctly claiming the invention, it is believed that the present invention will be better understood from the following description.

All percentages and ratios used hereinafter are by weight of total composition, unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

Citation of any reference herein is not an admission regarding any determination as to its availability as prior art to the claimed invention.

Herein, "comprising" means that other steps and other components which do not affect the end result can be added. This term encompasses the terms "consisting of" and "consisting essentially of."

The delivery systems herein are comprised of a delivery composition and a protective composition. The delivery and protective compositions herein comprise essential components, as well as optional components. The essential and optional components of these compositions of the present invention are described in the following paragraphs.

### DELIVERY COMPOSITION

The delivery composition herein is a composition for delivering an oral care substance to the oral cavity. The delivery composition comprises an oral care substance for providing a therapeutic, prophylactic, and/or cosmetic benefit to the oral cavity. One preferred embodiment of the delivery composition is comprised of an organosiloxane resin; a volatile carrier capable of solubilizing the organosiloxane resin; a rheology modifier; and at least one oral care substance.

Another preferred embodiment of the delivery composition is comprised of an organosiloxane resin; a fluid diorganopolysiloxane-based polymer; a volatile carrier capable of solubilizing the organosiloxane resin and the fluid diorganopolysiloxane-based polymer; a rheology modifier; and at least one oral care substance.

### PROTECTIVE COMPOSITION

The protective composition acts as a protective coating to prolong the presence of the oral care substance or substances contained in the delivery composition in the oral cavity. The protective composition need not comprise an oral care substance.

One preferred embodiment of the protective composition is comprised of an organosiloxane resin and a volatile carrier capable of solubilizing the organosiloxane resin. A rheology modifier may also be present.

Another preferred embodiment of the protective composition is comprised of an organosiloxane resin, a fluid diorganopolysiloxane-based polymer, a volatile carrier capable of solubilizing the organosiloxane resin and the fluid diorganopolysiloxane-based polymer. A rheology modifier may also be present.

The protective composition need not comprise an oral care substance in order to act as a protective coating for the delivery composition. However, it should be noted that any of the oral care substances described herein could additionally be added to the protective composition.

It should also be noted that more than one application of the protective composition, following the application of the delivery composition, can be made. Multiple applications of the protective composition can be made and are within the scope of the present invention.

### Organosiloxane Resins

Silicone resins are highly crosslinked polymeric siloxane systems. The crosslinking is introduced through the incorporation of tri-functional and tetra-functional silanes with mono-functional or di-functional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units, and hence, a sufficient level of crosslinking, such that they dry down to a rigid, or hard, film are considered to be silicone resins. The ratio of oxygen atoms to silicon atoms is indicative of the level of crosslinking in a particular silicone material. Silicone materials which have at least 1.1 oxygen atoms per silicon atom will generally be silicone resins herein. Preferably, the ratio of oxygen:silicon atoms is at least 1.2:1.0.

Silicone materials and silicone resins in particular can conveniently be identified according to a shorthand nomenclature system well known to those skilled in the art as the "MDTQ" nomenclature. Under this system, the silicone is described according to the presence of various siloxane monomer units which make up the silicone. Briefly, the symbol M denotes the mono-functional unit (CH₃)₃SiO)_{.5}; D denotes the difunctional unit (CH₃)₂SiO; T denotes the trifunctional unit (CH₃)SiO_{1.5}; and Q denotes the quadra- or tetra-functional unit SiO₂. Note that a small amount, up to about 5% of silanol or alkoxy functionality may also be present in the resin structure as a result of processing.

Primes of the unit symbols, e.g., M', D', T, and Q', denote substituents other than methyl, and must be specifically defined for each occurrence. Typical alternate substituents include groups such as vinyl, phenyl, amino, hydroxyl, etc. The molar ratios of the various units, either in terms of subscripts to the symbols indicating the total number of each type of unit in the silicone, or an average thereof, or as specifically indicated ratios in combination with molecular weight, complete the description of the silicone material under the MDTQ system. Higher relative molar amounts of T, Q, T' and/or Q' to D, D', M and/or M' in a silicone resin is indicative of higher levels of crosslinking. As discussed before, however, the overall level of crosslinking can also be indicated by the oxygen to silicon ratio.

The organosiloxane resins are solid at about 25°C and the average molecular weight of the resins is from 1,000 to 10,000. The resins are soluble in organic solvents such as toluene, xylene, isoparaffins, and cyclosiloxanes or the volatile carrier described below, indicating that the resin is not sufficiently crosslinked such that the resin is insoluble in the volatile carrier.

The silicone resins preferred for use herein are MQ, MT, MTQ, and MDTQ resins; such MQ resins are disclosed in U.S. Patent 5,330,747, Krzysik, issued July 19, 1994. Thus, the preferred silicone substituent is methyl. Especially preferred are MQ resins wherein the M:Q ratio is from 0.5:1.0 to 1.5:1.0. Organosiloxane resins such as these are commercially available, for example, Wacker 803 and 804 available from Wacker Silicones Corporation of Adrian, Michigan, US, and G.E. 1170-002 from the General Electric Company.

The level of the resin that is used in the compositions is dependent on its degree of solubility in the formulation, particularly in the solvents used. Generally, the range of resin used in the present invention is from 5% to 70%, preferably from 15% to 45%, and even more preferably from 20% to 40%.

### Fluid Diorganopolysiloxane-based Polymers

In addition to the organosiloxane resins disdosed above, the compositions of the present invention may further comprise a fluid diorganopolysiloxane-based polymer to be combined with the organosiloxane resins. Said fluid diorganopolysiloxane-based polymers useful in the present invention span a large range of viscosities; from 10 to 10,000,000 mm²S⁻¹ (centistokes (cSt)) at 25°C. Some diorganopolysiloxane polymers useful in this invention exhibit viscosities greater than 10,000,000 mm²S⁻¹ (centistokes (cSt)) at 25 °C and therefore are characterized by manufacturer specific penetration testing. Examples of this characterization are GE silicone materials SE 30 and SE 63 with penetration specifications of 500-1500 and 250-600 (tenths of a millimeter) respectively.

Among the fluid diorganopolysiloxane-based polymers of the present invention are diorganopolysiloxane polymers comprising repeating units, where said units correspond to the formula (R₂SiO)ₙ, where R is a monovalent hydrocarbon radical containing from 1 to 6 carbon atoms, preferably selected from the group consisting of methyl, ethyl, propyl, isopropyl, butyl, isobutyl, t-butyl, amyl, hexyl, vinyl, allyl, cyclohexyl, amino alkyl, phenyl, fluoroalkyl and mixtures thereof. The fluid diorganopolysiloxane polymers employed in the present invention may contain one or more of these hydrocarbon radicals as substituents on the siloxane polymer backbone. The fluid diorganopolysiloxane polymers may be terminated by triorganosilyl groups of the formula (R'₃Si) where R' is a radical selected from the group consisting of monovalent hydrocarbons containing from 1-6 carbon atoms, hydroxyl groups, alkoxyl groups and mixtures thereof. The fluid diorganopolysiloxane polymer must be compatible in solution with the organosiloxane resin and the volatile carrier. The term "compatible" refers to the formation of a single phase solution when the fluid diorganopolysiloxane polymer, the organosiloxane resin and the volatile carrier are mixed together in ratios required for a specific formulation. For example, the lower viscosity fluid diorganopolysiloxane polymers (viscosity of about 10 to mm²S⁻¹ (100cSt.)) are particularly useful when using ethanol as the principal volatile carrier. For higher viscosity polymers, e.g., poly(dimethylsiloxane) herein referred to as PDMS or silicone gum, having a viscosity of at least 100,000 mm²S⁻¹ (cSt,) volatile carriers other than ethanol are preferred. Silicone gum corresponds to the formula: 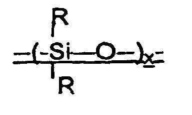 where R is a methyl group.

Fluid diorganopolysiloxane polymers such as these are commercially available, for example, SE 30 silicone gum and SF96 silicone fluid available from the General Electric Company. Similar materials can also be obtained from Dow Coming and from Wacker Silicones.

Another fluid diorganosiloxane-based polymer preferred for use in the present invention is a dimethicone copolyol to modify film forming characteristics as desired. The dimethicone copolyol can be further characterized as polyalkylene oxide modified polydimethysiloxanes, such as manufactured by the Witco Corporation under the trade name Silwet. Similar materials can be obtained from Dow Coming, Wacker Silicones and Goldschmidt Chemical Corporation as well as other silicone manufacturers.

In preferred embodiments of the present invention, the ratio of organosiloxane resin to fluid diorganopolysiloxane-based polymer is preferably from 15:1 to 1:15, more preferably from 2:1 to 8:1, and still more preferably from 4:1 to 6:1.

### Volatile Carriers

In the present invention, the organosiloxane resin and the fluid diorganosiloxane-based polymer above must be easily transferred to the oral cavity surfaces, such as to the tooth enamel. To achieve delivery, it is necessary that the resin or the resin/polymer combination above be incorporated into a carrier, specifically a volatile carrier which must quickly volatilize from the oral cavity surfaces, leaving a film on the application surfaces. The volatile carrier must solubilize the organosiloxane resin and if present in the composition, the fluid diorganosiloxane-based polymer.

The volatile carrier comprises from 10% to 90%, preferably from 15% to 80%, and more preferably from 20% to 70% of the composition. The volatile carrier of the present invention is selected from the group consisting of hydrocarbon oils, volatile silicones, non-hydrocarbon solvents, and mixtures thereof.

Hydrocarbon oils useful in the present invention include those having boiling points in the range of 60-260°C, more preferably hydrocarbon oils having from C₈ to C₂₀ chain lengths, most preferably C₈ to C₂₀ isoparaffins. Of these isoparaffins most preferred are selected from the group consisting of isododecane, isohexadecane, isoeocosane, 2,2,4-trimethylpentane, 2,3-dimethylhexane and mixtures thereof. Most preferred is isododecane, available as, for example, Permethyl 99A from Permethyl Corporation corresponding to the formula:

CH₃(CH₂)₁₀ CH₃

Preferred volatile silicone fluids indude cyclomethicones having 3, 4 and 5 membered ring structures corresponding to the formula: 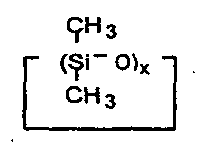 where X is from 3 to 6. Such volatile silicones include 244 Fluid, 344 Fluid and 245 Fluid, and 345 Fluid all from Dow Coming Corporation.

The general classes of non-hydrocarbon solvents useful herein include esters, ketones, alcohols, fluorocarbons and fluorocarbon ethers having boiling points in the range of 60 to 200 °C. Non-hydrocarbon solvents or mixtures thereof particularly useful include those that are capable of solubilizing the resin and/or the diorganopolysiloxane-based polymer. Such solvents include but are not limited to ethanol, acetone, butanone, ethyl acetate, propyl acetate, amyl acetate, ethyl butyrate, methyl nonafluoroisobutyl ether, methyl nonafluorobutyl ether, and mixtures thereof. These non-hydrocarbon solvents are readily available such as ethyl acetate and methyl ethyl ketone, both supplied by J. T. Baker of Phillispburg, N.J, and HFE (a mixture of methyl nonafluoroisobutyl ether and methyl nonafluorobutyl ether), supplied by the 3M Company.

### Rheology Modifiers

The compositions further comprise a rheology modifier which inhibits settling and separation of components or controls settling in a manner which facilitates re-dispersion and may control rheological flow properties. Suitable rheology modifiers herein include organo modified clays, silicas, polyethylene, and mixtures thereof. The preferred organophilic clays comprise quatemium-18 hectorite or Stearalkonium hectorite, such as Bentone 27 and 38™ from Rheox, organoclay dispersion such as Bentone ISD gel ™; or bentonite organo modified clays such as Bentone 34 ™ from Rheox or the Claytone Series ™ from Southern Clay Products; and mixtures thereof. The preferred silicas may be fumed silica such as the Aerosil ™ series from Degussa or the Cab-o-sil ™ series from Cabot Corporation, silica gels such as the Sylodent ™ or Sylox ™ series from W. R. Grace & Co. or precipitated silica such as Zeothix 265 from J. M. Huber Corporation.

The rheology modifier is preferably present in the composition at a level of from 0.1% to 30%, preferably from 0.5% to 10%, and even more preferably 1 % to 3% of the composition.

### Oral Care Substances

The oral care substance preferably contains an active at a level where upon directed use, the benefit sought by the wearer is promoted without detriment to the oral surface to which it is applied. Examples of the oral conditions these actives address include, but, are not limited to, appearance and structural changes to teeth, whitening, stain bleaching, stain removal, plaque removal, tartar removal, cavity prevention and treatment, inflamed and/or bleeding gums, mucosal wounds, lesions, ulcers, aphthous ulcers, cold sores, tooth abscesses, and the elimination of mouth malodor resulting from the conditions above and other causes such as microbial proliferation.

Suitable oral care substances include any material that is generally considered safe for use in the oral cavity and that provides changes to the overall appearance and/or health of the oral cavity. The level of oral care substance in the compositions of the present invention is generally, unless specifically noted, from 0.01% to 50%, preferably from 0.1% to 20%, more preferably from 0.5% to 10%, and even more preferably from 1% to 7%, by weight of the composition.

Oral care compositions or substances of the present invention may include many of the actives previously disclosed in the art. The following is a non-limiting list of oral care actives that may be used in the present invention.

### 1. Teeth Whitening Actives

Teeth whitening actives may be included in the oral care substance of the present invention. The actives suitable for whitening are selected from the group consisting of the peroxides, metal chlorites, perborates, percarbonates, peroxyacids, persulfates, and combinations thereof. Suitable peroxide compounds include hydrogen peroxide, urea peroxide, calcium peroxide, carbamide peroxide, and mixtures thereof. Most preferred is carbamide peroxide. Suitable metal chlorites include calcium chlorite, barium chlorite, magnesium chlorite, lithium chlorite, sodium chlorite, and potassium chlorite. Additional whitening actives may be hypochlorite and chlorine dioxide. The preferred chlorite is sodium chlorite. A preferred percarbonate is sodium percarbonate. Preferred persulfates are xones.

### 2. Anti-tartar Agents

Anti-tartar agents known for use in dental care products include phosphates. Phosphates include pyrophosphates, polyphosphates, polyphosphonates and mixtures thereof. Pyrophosphates are among the best known for use in dental care products. Pyrophosphate and polyphosphate ions are delivered to the teeth derive from pyrophosphate polyphosphate salts. The pyrophosphate salts useful in the present compositions include the dialkali metal pyrophosphate salts, tetra-alkali metal pyrophosphate salts, and mixtures thereof. Disodium dihydrogen pyrophosphate (Na₂H₂P₂O₇), tetrasodium pyrophosphate (Na₄P₂O₇), and tetrapotassium pyrophosphate (K₄P₂O₇) in their unhydrated as well as hydrated forms are the preferred species. While any of the above mentioned pyrophosphate salts may be used, tetrasodium pyrophosphate salt is preferred. Sodium polyphosphate and triethanolamine polyphosphates, for example, are preferred.

The pyrophosphate salts are described in more detail in Kirk & Othmer, *Encyclopedia of Chemical Technology,* Third Edition, Volume 17, Wiley-Interscience Publishers (1982). Additional anticalculus agents include pyrophosphates or polyphosphates disclosed in U.S. Patent No. 4,590,066 issued to Parran & Sakkab on May 20, 1986; polyacrylates and other polycarboxylates such as those disclosed in U.S. Patent No. 3,429,963 issued to Shedlovsky on February 25, 1969 and U.S. Patent No. 4,304,766 issued to Chang on December 8, 1981; and U.S. Patent No. 4,661,341 issued to Benedict & Sunberg on April 28, 1987; polyepoxysuccinates such as those disclosed in U.S. Patent No. 4,846,650 issued to Benedict, Bush & Sunberg on July 11, 1989; ethylenediaminetetraacetic acid as disclosed in British Patent No. 490,384 dated February 15, 1937; nitrilotriacetic acid and related compounds as disclosed in U.S. Patent No. 3,678,154 issued to Widder & Briner on July 18, 1972; polyphosphonates as disclosed in U.S. Patent No. 3,737,533 issued to Francis on June 5, 1973, U.S. Patent No. 3,988,443 issued to Ploger, Schmidt-Dunker & Gloxhuber on October 26, 1976 and U.S. Patent No. 4,877,603 issued to Degenhardt & Kozikowski on October 31, 1989. Anticalculus phosphates include potassium and sodium pyrophosphates; sodium tripolyphosphate; diphosphonates, such as ethane-1-hydroxy-1,1-diphosphonate, 1-azacycloheptane-1,1-diphosphonate, and linear alkyl diphosphonates; linear carboxylic acids; and sodium zinc citrate.

Agents that may be used in place of or in combination with the pyrophosphate salt include such known materials as synthetic anionic polymers including polyacrylates and copolymers of maleic anhydride or acid and methyl vinyl ether (e.g., Gantrez), as described, for example, in U.S. Patent 4,627,977, to Gaffar et al.; as well as, e.g., polyamino propoane sulfonic acid (AMPS), zinc citrate trihydrate, polyphosphates (e.g., tripolyphosphate; hexametaphosphate), diphosphonates (e.g., EHDP; AHP), polypeptides (such as polyaspartic and polyglutamic acids), and mixtures thereof.

### 3. Fluoride Ion Source

Fluoride ion sources are well known for use in oral care compositions as anticaries agents. Fluoride ions are contained in a number of oral care compositions for this purpose, particularly toothpastes. Patents disclosing such toothpastes include U.S. Pat. No. 3,538,230, Nov. 3, 1970 to Pader et al; U.S. Pat. No. 3,689,637, Sept. 5, 1972 to Pader; U.S. Pat. No. 3,711,604, Jan 16, 1973 to Colodney et al; U.S. Pat. No. 3,911,104, Oct. 7, 1975 to Harrison; U.S. Pat. No. 3,935,306, Jan. 27, 1976 to Roberts et al; and U.S. Pat. No. 4,040,858, Aug. 9, 1977 to Wason.

Application of fluoride ions to dental enamel serves to protect teeth against decay. A wide variety of fluoride ion-yielding materials can be employed as sources of soluble fluoride in the instant compositions. Examples of suitable fluoride ion-yielding materials are found in Briner et al; U.S. Pat. No. 3,535,421; issued Oct. 20, 1970 and Widder.et al; U.S. Pat. No. 3,678,154; issued July 18, 1972. Preferred fluoride ion sources for use herein include sodium fluoride, potassium fluoride and ammonium fluoride. Sodium fluoride is particularly preferred. Preferably the instant compositions provide from 50 ppm to 10,000 ppm, more preferably from 100 to 3000 ppm, of fluoride ions in the compositions that contact dental surfaces when used with the delivery system of the present invention.

### 4. Anti-microbial Agents

Anti-microbial agents can also be present in the oral care compositions or substances of the present invention. Such agents may include, but are not limited to, 5-chloro-2-(2,4-dichlorophenoxy)-phenol, commonly referred to as triclosan, and described in The Merck Index, 11th ed. (1989), pp. 1529 (entry no. 9573) in U.S. Patent No. 3,506,720, and in European Patent Application No. 0,251,591 of Beecham Group, PLC, published January 7, 1988; phthalic acid and its salts including, but not limited to those disclosed in U.S. Pat. 4,994,262, Feb. 19, 1991, preferably magnesium monopotassium phthalate, chlorhexidine (Merck Index, no. 2090), alexidine (Merck Index, no. 222; hexetidme (Merck Index, no. 4624); sanguinarine (Merck Index, no. 8320); benzalkonium chloride (Merck Index, no. 1066); salicylanilide (Merck Index, no. 8299); domiphen bromide (Merck Index, no. 3411); cetylpyridinium chloride (CPC) (Merck Index, no. 2024; tetradecylpyridinium chloride (TPC); N-tetradecyl-4-ethylpyridinium chloride (TDEPC); octenidine; delmopinol, octapinol, and other piperidino derivatives; nicin preparations; zinc/stannous ion agents; antibiotics such as augmentin, amoxicillin, tetracycline, doxycycline, minocycline, and metronidazole; and analogs and salts of the above; essential oils including thymol, geraniol, carvacrol, citral, hinokitiol, eucalyptol, catechol (particularly 4-allyl catechol) and mixtures thereof; methyl salicylate; hydrogen peroxide; metal salts of chlorite and mixtures of all of the above.

### 5. Anti-inflammatory Agents

Anti-inflammatory agents can also be present in the oral care compositions or substances of the present invention. Such agents may include, but are not limited to, non-steroidal anti-inflammatory agents or NSAIDs such as ketorolac, flurbiprofen, ibuprofen, naproxen, indomethacin, aspirin, ketoprofen, piroxicam and meclofenamic acid. Use of NSAIDs such as Ketorolac are claimed in U.S. Patent 5,626,838, issued May 6, 1997. Disclosed therein are methods of preventing and, or treating primary and reoccurring squamous cell carcinoma of the oral cavity or oropharynx by topical administration to the oral cavity or oropharynx an effective amount of an NSAID.

### 6. Nutrients

Nutrients may improve the condition of the oral cavity and can be included in the oral care compositions or substances of the present invention. Nutrients include minerals, vitamins, oral nutritional supplements, enteral nutritional supplements, and mixtures thereof.

Minerals that can be included with the compositions of the present invention include calcium, phosphorus, fluoride, zinc, manganese, potassium and mixtures thereof. These minerals are disclosed in Drug Facts and Comparisons (loose leaf drug information service), Wolters Kluer Company, St. Louis, Mo., ©1997, pp10-17.

Vitamins can be included with minerals or used separately. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Such vitamins are disclosed in Drug Facts and Comparisons (loose leaf drug information service), Wolters Kluer Company, St. Louis, Mo., ©1997, pp. 3-10.

Oral nutritional supplements include amino acids, lipotropics, fish oil, and mixtures thereof, as disclosed in Drug Facts and Comparisons (loose leaf drug information service), Wolters Kluer Company, St. Louis, Mo., ©1997, pp. 54-54e. Amino acids include, but, are not limited to L-Tryptophan, L-Lysine, Methionine, Threonine, Levocarnitine or L- carnitine and mixtures thereof. Lipotropics include, but, are not limited to choline, inositol, betaine, linoleic acid, linolenic acid, and mixtures thereof. Fish oil contains large amounts of Omega-3 (N-3) Polyunsaturated fatty acids, eicosapentaenoic acid and docosahexaenoic acid.

Entenal nutritional supplements include, but, are not limited to protein products, glucose polymers, corn oil, safflower oil, medium chain triglycerides as disclosed in Drug Facts and Comparisons (loose leaf drug information service), Wolters Kluer Company, St. Louis, Mo., ©1997, pp. 55-57.

### 7. Mouth and Throat Products

Other materials that can be used with the present invention include commonly known mouth and throat products. Such products are disclosed in Drug Facts and Comparisons (loose leaf drug information service), Wolters Kluer Company, St. Louis, Mo., ©1997, pp. 520b-527. These products include, but, are not limited to anti-fungal, antibiotic and analgesic agents.

### 8. Antioxidants

Antioxidants are generally recognized as useful in compositions such as those of the present invention. Antioxidants are disclosed in texts such as Cadenas and Packer, The Handbook of Antioxidants, © 1996 by Marcel Dekker, Inc. Antioxidants that may be included in the oral care composition or substance of the present invention include, but are not limited to Vitamin E, ascorbic acid, Uric acid, carotenoids, Vitamin A, flavonoids and polyphenols, herbal antioxidants, melatonin, aminoindoles, lipoic acids and mixtures thereof.

### 9. H-2 Antagonists

Histamine-2 (H-2 or H2) receptor antagonist compounds (H-2 antagonists) may be used in the oral care composition of the present invention. As used herein, selective H-2 antagonists are compounds that block H-2 receptors, but do not have meaningful activity in blocking histamine-1 (H-1 or H1) receptors. Selective H-2 antagonists stimulates the contraction of smooth muscle from various organs, such as the gut and bronchi; this effect can be suppressed by low concentrations of mepyramine - a typical antihistaminic drug. The pharmacological receptors involved in these mepyramine-sensitive histamine responses have been defined as H-1 receptors (Ash, A.S.F. & H.O. Schild, Brit. J. Pharmacol Chemother., Vol. 27 (1966), p. 427. Histamine also stimulates the secretion of acid by the stomach (Loew, E.R. & O. Chickering, Proc. Soc. Exp. Biol. Med., Vol. 48 (1941), p. 65), increases the heart rate (Trendelenburg, U., J. Pharmacol., Vol. 130 (1960), p. 450), and inhibits contractions in the rat uterus (Dews, P.B. & J.D.P. Graham, Brit. J. Pharmacol. Chemother., Vol. 1 (1946), p. 278); these actions cannot be antagonized by mepyramine and related drugs. The H-2 antagonists useful in the oral care compositions or substances are those that blockade the receptors involved in mepyramine-insensitive, non-H-1 (H-2), histamine responses, and do not blockade the receptors involved in mepyramine-sensitive histamine responses.

Selective H-2 antagonists are those compounds found to be H-2 antagonists through their performance in classical preclinical screening tests for H-2 antagonist function. Selective H-2 antagonists are identified as compounds which can be demonstrated to function as competitive or non-competitive inhibitors of histamine-mediated effects in those screening models specifically dependent upon H-2 receptor function, but to lack significant histamine antagonist activity in those screening models dependent upon H-1 receptor function. Specifically, this includes compounds that would be classified as described by Black, J.W., W.A.M. Duncan, C.J. Durant, C.R. Ganellin & E.M. Parsons, "Definition and Antagonism of Histamine H2-Receptors", Nature, Vol. 236 (April 21, 1972), pp. 385-390 (Black), as H-2 antagonists if assessed as described by Black through testing with the guinea pig spontaneously beating right atria in vitro assay and the rat gastric acid secretion in vivo assay, but shown to lack in significant H-1 antagonist activity relative to H-2 antagonist activity, if assessed as described by Black with either the guinea pig ileum contraction in vitro assay or the rat stomach muscle contraction in vivo assay. Preferably selective H-2 antagonists demonstrate no significant H-1 activity at reasonable dosage levels in the above H-1 assays. Typical reasonable dosage level is the lowest dosage level at which 90% inhibition of histamine, preferably 99% inhibition of histamine, is achieved in the above H-2 assays.

Selective H-2 antagonists include compounds meeting the above criteria which are disclosed in U.S. Patents 5,294,433 and 5,364,616 Singer et al., issued 3/15/94 and 11/15/94 respectively and assigned to Procter & Gamble, wherein the selective H-2 antagonist is selected from the group consisting of cimetidine, etintidine, ranitidine, ICIA-5165, tiotidine, ORF-17578, lupitidine, donetidine, famotidine, roxatidine, pifatidine, lamtidine, BL-6548, BMY-25271, zaltidine, nizatidine, mifentidine, BMY-52368 (SKF-94482), BL-6341A, ICI-162846, ramixotidine, Wy-45727, SR-58042, BMY-25405, loxtidine, DA-4634, bisfentidine, sufotidine, ebrotidine, HE-30-256, D-16637, FRG-8813, FRG-8701, impromidine, L-643728, and HB-408. 4. Particularly preferred is cimetidine (SKF-92334), N-cyano-N'-methyl-N"-(2-(((5-methyl-1H-imidazol-4-yl)methyl)thio)ethyl)guanidine:

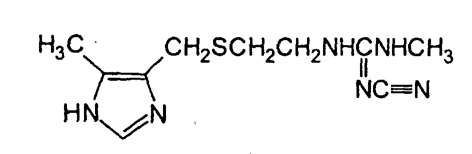

Cimetidine is also disclosed in the Merck Index, 11th edition (1989), p. 354 (entry no. 2279), and Physicians' Desk Reference, 46th edition (1992), p. 2228. Related preferred H-2 antagonists include burimamide and metiamide.

### 10. Analgesic Actives

Anti-pain or desensitizing agents can also be present in the oral care compositions or substances of the present invention. Such agents may include, but are not limited to, strontium chloride, potassium nitrate, natural herbs such as gall nut, Asarum, Cubebin, Galanga, scutellaria, Liangmianzhen, Baizhi, etc.

### 11. Anti-viral Actives

Antiviral actives useful in the present composition include any know actives that are routinely use to treat viral infections. Such anti-viral actives are disdosed in Drug Facts and Comparisons (loose leaf drug information service), Wolters Kluer Company, St. Louis, Mo., ©1997, pp. 402(a)-407(z). Specific examples include anti-viral actives disclosed in U.S. Patent 5,747,070, issued May 5, 1998 to Satyanarayana Majeti. Said Patent discloses the use of stannous salts to control viruses. Stannous salts and other anti-viral actives are described in detail in Kirk & Othmer, Encyclopedia of Chemical Technology, Third Edition, Volume 23, Wiley-Interscience Publishers (1982), pp. 42-71. The stannous salts that may be used in the present invention would include organic stannous carboxylates and inorganic stannous halides. While stannous fluoride may be used, it is typically used only in combination with another stannous halide or one or more stannous carboxylates or another therapeutic agent.

### 12. Other Ingredients

In addition to the above materials of the composition of the present invention, a number of other components may desirably be added to the oral care substance. Additional components include, but are not limited to, flavoring agents, sweetening agents, xylitol, opacifiers, coloring agents, surfactants, and chelants such as ethylenediaminetetraacetic acid. Suitable flavoring agents include, but are not limited to, oil of peppermint, oil of sassafras, dove bud oil, peppermint, menthol, anethole, thymol, methyl salicylate, eucalyptol, cassia, 1-menthyl acetate, sage, eugenol, parsley oil, oxanone, oil of wintergreen, alpha-irisone, oil of spearmint, marjoram, lemon, orange, propenyl guaethol, cinnamon, and mixtures thereof.

Pigments may also added to the compositions herein to more precisely indicate the locations at which the composition has actually been applied, allowing the user to apply the composition more thoroughly or evenly. However, such pigments are not intended to mask stains that may exist on the tooth surfaces.

These additional ingredients can also be used in place of the compounds disclosed above.

### EXAMPLES

The following examples further describe and demonstrate embodiments within the scope of the present invention. The examples are given solely for the purpose of illustration and are not to be construed as limitations of the present invention, as many variations thereof are possible.

**Table 1:**

| Hydrophobic Delivery Composition | | | | | | |
|---|---|---|---|---|---|---|
| Component | Ex.1 | Ex.2 | Ex.3 | Ex.4 | Ex.5 | Ex.6 |
| Organosiloxane | 25 | 25 | 24.6 | 25 | 25 | 25 |
| Resin¹ | | | | | | |
| Silicone Gum² | 12.5 | 4.2 | 3.5 | 2.5 | 1.8 | -- |
| Oral Care- | 17 | 17 | 17 | 17 | 17 | 17 |
| Substance³ | | | | | | |
| Volatile Carrier⁴ | 44.5 | 52.8 | 53.9 | 54.5 | 55.2 | 57.0 |
| Bentone Clay⁵ | 1 | 1 | 1 | 1 | 1 | 1 |
| TOTAL | 100 | 100 | 100 | 100 | 100 | 100 |

| | | | | | | |
|---|---|---|---|---|---|---|
| 1. E.g., MQ resin available as 1170-002 from General Electric. | | | | | | |
| 2. E.g., Dimethicone gum available as SE 30 or SE 63 from General Electric. | | | | | | |
| 3. E.g., Sodium percarbonate as a dry powder. | | | | | | |
| 4. E.g., Isododecane or a 50/50 mixture of ethyl acetate and butanone or a mixture of ethyl acetate, propyl acetate and HFE. The percentage of each individual solvent component in the mixed solvent systems can vary from 0 to 100%, respectively. | | | | | | |
| 5. Bentone 27 available from Rheox. | | | | | | |

**Table 2:**

| Hydrophobic Delivery Composition | | | | |
|---|---|---|---|---|
| Component | Ex.1 | Ex.2 | Ex.3 | Ex.4 |
| Organosiloxane Resin¹ | 33.43 | 33.43 | 35.14 | 33.43 |
| Silicone Gum² | 5.57 | 5.57 | 5.86 | 5.57 |
| Oral Care Substance³ | 19.00 | 12.00 | 19.00 | 19.00 |
| Ethyl acetate | 8.00 | 8.50 | 8.00 | 8.00 |
| Bentone Gel⁴ | 10.00 | 10.00 | 10.00 | 10.00 |
| DC-200/350cst.⁵ | 1.00 | 1.00 | 2.00 | 1.00 |
| HFE-7100⁶ | 21.00 | 26.00 | - | 19.50 |
| N-propyl acetate | 2.00 | 2.00 | - | 2.00 |
| 2-butanone | - | - | 19.00 | - |
| Aerosil 200⁷ (SiO₂) | - | - | 1.00 | - |
| Flavor | - | 1.50 | - | 1.50 |
| TOTAL | 100 | 100 | 100 | 100 |

| | | | | |
|---|---|---|---|---|
| 1. E.g., MQ resin available as 1170-002 from General Electric. | | | | |
| 2. E.g., Dimethicone gum available as SE 30 from General Electric. | | | | |
| 3. E.g., Sodium percarbonate as a dry powder. | | | | |
| 4. Bentone Gel IPM available from Rheox. | | | | |
| 5. DC-200/350 is: dimethylpolysiloxane, CAS# 9016-00-6, from Dow Coming. | | | | |
| 6. HFE-7100 is a mixture of Methyl Nonafluoroisobutyl Ether, CAS# 163702-087 and Methyl Nonafluorobutyl Ether, CAS# 163702-07-6, manufactured by 3M Co. | | | | |
| 7. Aerosil 200 is silicon dioxide (chemically prepared), CAS# 112945-52-5, from Degussa AG. | | | | |

### Method of Preparation

The delivery compositions of Tables 1 and 2 are non-aqueous. The oral care substances are dispersed or dissolved in a solution comprising the organosiloxane resin, the fluid diorganopolysiloxane-based polymer, the volatile carrier, and the rheology modifier.

The compositions of Tables 1 and 2 are suitably prepared as follows. Three hundred (300) grams of organosiloxane resin solution (for example, 43.7% MQ resin solution in isododecane or in a 50/50 mixture of ethyl acetate and butanone (or in a mixture of ethyl acetate, propyl acetate and HFE) are mixed with 147.30 grams of fluid diorganopolysiloxane polymer solution (for example, 50% SE30 silicone gum solution in isododecane or a 50/50 mixture of ethyl acetate and butanone or a mixture of ethyl acetate, propyl acetate and HFE). The oral care substances are then dispersed in the resin/gum mixture. This method may be carried out without the presence of the silicone gum.

All oral care substances described herein can be formulated as described above.

**Table 3:**

| Whitening Delivery Compositions | | | | | | |
|---|---|---|---|---|---|---|
| Component | Ex.1 | Ex.2 | Ex.3 | Ex.4 | Ex.5 | Ex.6 |
| Ethyl Acetate | 18.00 | 14.85 | 22.25 | 20.88 | 18.96 | 18.00 |
| 2-Butanone | 18.00 | 13.00 | 13.10 | 20.88 | 10.00 | 18.00 |
| Isododecane | -- | 10.00 | -- | -- | 11.54 | -- |
| Limonene | -- | 4.35 | -- | -- | 5.00 | -- |
| MQ Resin | 28.00 | 32.50 | 26.50 | 27.33 | 36.00 | 31.50 |
| SE 30 Silicon Gum | 7.00 | -- | 8.80 | 13.67 | -- | -- |
| Silicone Visc-100M | -- | -- | -- | -- | -- | 3.50 |
| Silicone Fluid 10 (cStk)mm²s⁻¹ | -- | 6.50 | -- | -- | 9.00 | -- |
| Bentone Gel ISD | 10.00 | -- | 6.40 | 9.95 | -- | 10.00 |
| Claytone HY | -- | 2.45 | -- | -- | 3.00 | -- |
| Cab-o-Sil | -- | -- | 1.50 | -- | -- | -- |
| Sodium Percarbonate | 19.00 | -- | 19.00 | 7.00 | -- | 19.00 |
| Carbamide Peroxide | -- | 15.00 | -- | -- | 5.00 | -- |
| Bismuth Oxychloride | -- | 1.15 | -- | -- | -- | -- |
| Titanium Dioxide | -- | -- | 1.00 | -- | 1.50 | -- |
| Flavor Oil | -- | -- | 0.15 | -- | -- | -- |
| Sodium Fluoride | -- | -- | 1.00 | -- | -- | -- |
| Sodium Saccharin | -- | 0.20 | 0.30 | 0.30 | -- | -- |
| TOTAL | 100.00 | 100.00 | 100.00 | 100.00 | 100.00 | 100.00 |

**Table 4:**

| Oral Care Delivery Compositions | | | | | |
|---|---|---|---|---|---|
| Component | Ex.1 | Ex.2 | Ex.3 | Ex.4 | Ex.5 |
| Ethyl Acetate | 24.50 | 27.75 | 22.00 | 19.96 | 21.10 |
| 2-Butanone | 24.50 | 16.30 | 22.00 | 10.00 | 21.10 |
| Isododecane | -- | -- | -- | 11.54 | -- |
| Umonene | -- | -- | -- | 5.00 | -- |
| MQ Resin | 30.40 | 33.00 | 28.80 | 36.00 | 32.84 |
| SE 30 Silicone Gum | 7.60 | 11.00 | 14.40 | -- | 8.21 |
| Silicone Fluid 10 (cStk)mm²S⁻¹ | -- | -- | -- | 9.00 | -- |
| Bentone Gel ISD | 10.00 | 8.00 | 10.50 | -- | 11.75 |
| Claytone HY | -- | -- | -- | 3.00 | -- |
| Cab-o-Sil | -- | 1.50 | -- | -- | -- |
| Bismuth Oxychloride | -- | 1.00 | -- | -- | -- |
| Titanium Dioxide | -- | -- | -- | 2.00 | -- |
| Flavor Oil | -- | 0.15 | -- | -- | -- |
| Potassium Nitrate | -- | -- | -- | -- | 5.00 |
| Sodium Chlorite | 3.00 | -- | -- | -- | -- |
| Tripolyphosphate | -- | -- | -- | 2.50 | -- |
| Sodium Fluoride | -- | 1.00 | -- | 1.00 | -- |
| Chlorhexidine Gluconate | -- | -- | 2.00 | -- | -- |
| Sodium Saccharin | -- | 0.30 | 0.30 | -- | -- |
| TOTAL | 100.00 | 100.00 | 100.00 | 100.00 | 100.00 |

**Table 5: Oral Care Delivery Compositions**

| Component | Ex.1 | Ex.2 | Ex.3 | Ex.4 | Ex.5 | Ex.6 |
|---|---|---|---|---|---|---|
| Ethyl Acetate | 26.00 | 22.00 | 35.00 | 28.00 | 25.77 | 23.00 |
| 2-Butanone | 25.95 | 17.00 | 9.00 | 14.50 | 25.50 | 23.45 |
| Isododecane | -- | 10.00 | -- | -- | 5.00 | -- |
| Limonene | -- | 4.00 | -- | -- | -- | -- |
| MQ Resin | 28.00 | 32.50 | 33.00 | 35.00 | 27.00 | 28.00 |
| SE 30 Silicone Gum | 7.00 | - | 11.00 | 5.00 | 3.00 | 7.00 |
| Silicone Fluid 10 (cStk)mm²S⁻¹ | -- | 6.50 | -- | -- | -- | -- |
| Bentone Gel ISD | 10.00 | -- | 8.00 | 10.00 | 10.00 | - |
| Claytone HY | -- | 2.00 | -- | -- | -- | 7.00 |
| Cab-o-Sil M7D | -- | -- | 1.50 | -- | -- | -- |
| Bismuth Oxychloride | -- | 5.00 | -- | -- | -- | 10.00 |
| Titanium Dioxide | 3.00 | -- | 1.00 | 5.00 | 2.00 | -- |
| Flavor Oil | -- | -- | 0.10 | -- | 0.15 | 0.15 |
| Polymethylsilsesquio xane | -- | -- | 1.00 | -- | -- | -- |
| Polymethylmethyacry late | 3.00 | 1.00 | -- | -- | 0.50 | -- |
| Nylon 12 | -- | -- | -- | 2.00 | -- | 1.00 |
| Silica | -- | -- | -- | -- | 0.50 | -- |
| FD&C Yellow #5 Al Lake | 0.05 | -- | 0.10 | -- | 0.05 | 0.10 |
| Iron Oxide, Red | -- | -- | -- | 0.50 | 0.03 | -- |
| Sodium Saccharin | -- | -- | 0.30 | -- | 0.50 | 0.30 |
| TOTAL | 100.00 | 100.00 | 100.00 | 100.00 | 100.00 | 100.00 |

**Table 6:**

| Ethanol Based Delivery Compositions | | | | | | |
|---|---|---|---|---|---|---|
| Component | Ex.1 | Ex.2 | Ex.3 | Ex.4 | Ex.5 | Ex.6 |
| Ethanol | 20.90 | 35.80 | 41.70 | 25.89 | 23.10 | 23.55 |
| Ethyl Acetate | -- | -- | -- | -- | 4.95 | -- |
| Ethyl Butyrate | 10.30 | -- | -- | 12.74 | 11.75 | 10.9 |
| Isoamyl Acetate | -- | -- | -- | -- | -- | 2.65 |
| Isododecane | -- | 13.00 | -- | -- | -- | -- |
| MQ Resin | 35.10 | 32.85 | 42.75 | 47.00 | 42.00 | 41.80 |
| Silicone Visc-100M | -- | -- | -- | -- | 7.15 | -- |
| Silicone Fluid 100 (cSt)mm²S⁻¹ | 7.80 | -- | -- | 9.65 | -- | 8.55 |
| Silicone Fluid 10 (cStk)mm²S⁻¹ | 3.90 | -- | 11.40 | -- | 4.00 | -- |
| Silwet L-7500 | -- | -- | -- | -- | -- | 6.58 |
| Bentone 27 | 1.50 | -- | 2.00 | 1.85 | 1.35 | 1.97 |
| Claytone APA | -- | 2.00 | -- | -- | -- | -- |
| Cab-o-Sil | -- | -- | -- | -- | 0.45 | -- |
| Sodium Percarbonate | 19.00 | -- | -- | -- | -- | -- |
| Carbamide Peroxide | -- | 15.00 | -- | -- | -- | -- |
| Potassium Nitrate | -- | -- | -- | -- | 5.00 | -- |
| Tripalyphosphate | -- | -- | -- | -- | -- | 2.50 |
| Chlorhexidine Digluconate | -- | -- | -- | 2.57 | -- | -- |
| Titanium Dioxide | 1.50 | -- | 2.00 | -- | -- | 1.50 |
| Flavor Oil | -- | -- | 0.05 | -- | -- | -- |
| Sodium Fluoride | -- | -- | 0.10 | -- | 0.25 | -- |
| Sodium Saccharin | -- | 0.20 | -- | 0.30 | -- | -- |
| | | | | | | |
| | 100.00 | 100.00 | 100.00 | 100.00 | 100.00 | 100.00 |

### Method of Preparation

The delivery compositions of Tables 3-6 are suitably prepared as follows. Add the solvents into a container suitable to minimize solvent loss. Add the rheology modifiers and mix until well dispersed. Add the silicone resin and mix until completely dissolved. Add the silicone gum and/or silicone fluids and mix until completely dissolved. At this time add any salts such as sodium percarbonate and/or other oral care actives, aesthetic ingredients such as opacifiers, sweeteners, dyes, and flavors. Continue mixing until homogeneous. Additional high shear mixing may be used to promote the mixing. Pack into airtight containers.

Alternatively premixes of the silicone resin and/or the silicone gum may be prepared prior to incorporation into the final blending step to facilitate silicone dissolution and ease of manufacturing. Depending on the formula composition, the order of ingredient addition may also vary such as the addition of the rheology modifier(s) may be moved to a later step allowing lower viscosity to be maintained until the later stages of the blending step.

The embodiments disclosed and represented by the examples of the previous Tables have many advantages. For example, they provide better durability and subsequent delivery of oral care substances, particularly to the surfaces of the teeth. They also provide a convenient, discrete, and easy to use product form which can deliver benefits that are significantly different from those that can be achieved by conventional product forms. In addition, oral care actives that would exhibit instability in an aqueous-based film system can be incorporated into the compositions herein without compromising stability.

**Table 7:**

| Protective Composition | | | | | | |
|---|---|---|---|---|---|---|
| Component | Ex.1 | Ex.2 | Ex.3 | Ex.4 | Ex.5 | Ex.6 |
| Organosiloxane Resin¹ | 36.3 | 39.5 | 39.5 | 40.5 | 41 | 42 |
| Silicon Gum² | 18.2 | 6.7 | 5.6 | 4.0 | 2.8 | - |
| Volatile Carrier³ | 44.5 | 52.8 | 53.9 | 54.5 | 55.2 | 57.0 |
| Bentone Clay⁴ | 1 | 1 | 1 | 1 | 1 | 1 |
| | 100 | 100 | 100 | 100 | 100 | 100 |

| | | | | | | |
|---|---|---|---|---|---|---|
| 1. E.g., MQ resin available as 1170-002 from General Electric. | | | | | | |
| 2. E.g., Dimethicone gum (2,500,000 mm²S⁻¹ (cst)) available as SE63 from General Electric. | | | | | | |
| 3. E.g., Isododecane or a 50/50 mixture of ethyl acetate and butanone. The percentage of ethyl acetate and butanone in the mixed system can vary from 0 to 100%, respectivety; or a mixture of HFE, propyl acetate, and ethyl acetate. | | | | | | |
| 4. E.g., Bentone 27 available from Rheox. | | | | | | |

### Method of Use

In practicing the system of the present invention, the user need only apply a delivery composition that contains the oral care substance or substances necessary in order to obtain a desired effect, e.g., whitening, breath freshening, caries prevention, pain relief, gum health, tartar control, etc., followed by a protective composition to the tooth surfaces in the areas desired. The compositions herein may also be applied to other surfaces of the oral cavity, such as the gingival or mucosal tissues, or to any other oral cavity surface. The compositions herein can be applied with a brush, a pen applicator, a doe's foot applicator, or the like, or even with the fingers.

A film containing the oral care substance quickly forms on the surface to which the delivery composition has been applied. Then, the user applies the protective composition to the same surfaces, on top of the delivery composition. Similarly, the protective composition quickly forms a film.

Prolonged delivery of the oral care substance is made possible as the oral care substance is released from the film formed by the delivery composition over time. The protective composition protects the delivery composition from wearing off to prolong the wear of the delivery compositions and therefore to prolong the presence of the oral care substance in the oral cavity. In addition, the protective compositions force the oral care substance being released to act on the applied area, and inhibit it from being released elsewhere in the oral cavity or from being worn away by saliva, eating, etc.

Without being bound by theory, it is believed that the protection efficacy provided by the protective composition depends on the amount of resin present, as well as the resin to polymer ratio and the polymer concentration in those compositions further comprising a fluid diorganopolysiloxane-based polymer. It is further believed that the protection efficacy is influenced by the specific oral care substance that is present in the delivery composition.

Then, any residual oral care substance or film may be easily removed by wiping, brushing or rinsing the oral surface after a desired period of time has elapsed, or in the normal course of tooth brushing or other oral care activities. Without being bound by theory, it is believed that the wear time of the systems herein is from about 2 hours to 10 hours, regardless of the reactivity of the oral care substance. Preferably, the systems are almost unnoticeable when applied to the oral cavity.

It is not necessary to prepare the oral cavity before using the system of the present invention. For example, the user may or may not choose to brush the teeth or rinse the mouth before applying the compositions. The surfaces of the oral cavity are neither required to be dried nor to be excessively wet with saliva or water before the compositions are applied. However, it is believed that adhesion to the tooth enamel surfaces will be improved if the teeth are dry when the compositions are applied.

It should be understood that the present invention is useful not only for delivering an oral care substance to the oral cavity of a human, but also for delivering an oral care substance to the oral cavity of an animal, e.g., household pets or other domestic animals, or animals kept in captivity.

## Claims

1. A system for delivering an oral care substance to the oral cavity, the system being suitable for self-treatment and home use by a user and comprising:
a) a delivery composition comprised of:
i) an organosiloxane resin;
ii) a volatile carrier capable of solubilizing the organosiloxane resin;
iii) a rheology modifier;
iv) and at least one oral care substance; and
b) a protective composition comprised of:
i) an organosiloxane resin; and
ii) a volatile carrier capable of solubilizing the organosiloxane resin.

2. The system according to Claim 1 wherein the delivery composition further comprises a fluid diorganopolysiloxane-based polymer and the volatile carrier is capable of solubilizing the fluid diorganopolysiloxane-based polymer.

3. The system of Claim 1 and 2 wherein the protective composition further comprises a fluid diorganopolysiloxane-based polymer.

4. The system of Claim 2 or 3 wherein the fluid diorganopolysiloxane-based polymer comprises repeating units of the formula (R₂SiO)ₙ, where R is a monovalent hydrocarbon radical group containing from 1 to 6 carbon atoms.

5. The system of Claim 4 wherein said hydrocarbon radical group is selected from the group consisting of methyl, ethyl, propyl, isopropyl, butyl, isobutyl, t-butyl, amyl, hexyl, vinyl, allyl, cyclohexyl, amino alkyl, phenyl, fluoroalkyl and mixture thereof.

6. The system of Claim 5 wherein the fluid diorganopolysiloxane based polymer is terminated by triorganosilyl groups of the formula (R'₃Si) where R' is a monovalent hydrocarbon radical selected from the group consisting of 1 to 6 carbon atoms, hydroxyl groups, alkoxl groups and mixtures thereof.

7. The system of Claim 6 wherein the fluid diorganopolysiloxane-based polymer is poly(dimethylsiloxane).

8. The system of Claim 1 wherein the organosiloxane resin in the delivery and protective compositions is present at a level of from 5% to 70%.

9. The system of Claim 8 wherein the organosiloxane resin is selected from the group consisting of (CH₃)₃SiO)_{0.5} "M" units, (CH₃)₂SiO "D" units, (CH₃)SiO_{1.5} "T" units, SiO₂ "Q" units, and mixtures thereof.

10. The system of Claim 2 or Claim 3 wherein the ratio of organosiloxane resin to fluid diorganopolysiloxane-based polymer in the delivery and protective compositions is from 15:1 to 1:15.

11. The system of Claim 9 wherein the organosiloxane resin is comprised of MQ units, wherein the M:Q ratio is from 0.5:1.0 to 1.5:1.0.

12. The system of any of Claims 1 to 3 wherein the volatile carrier in the delivery and protective compositions is selected from the group consisting of hydrocarbon oils, volatile silicones, non-hydrocarbon solvents, and mixtures thereof.

13. The system of Claim 12 wherein the volatile carrier is selected from the group consisting of ethanol, isododecane, butanone, ethyl acetate, propyl acetate, methyl nonafluoroisobutyl ether, methyl nonafluorobutyl ether and mixtures thereof.

14. The system of any of Claims 1 to 3 wherein the protective composition further comprises a rheology modifier.

15. The system of Claim 14 wherein the rheology modifier in the delivery and protective compositions is selected from the group consisting of organo modified clays, silicas, polyethylene, and mixtures thereof.

16. The system of Claim 15 wherein the rheology modifier is present in the delivery and protective compositions at a level of from 0.1% to 30%.

17. The system of Claim 1 or Claim 2 wherein the oral care substance in the delivery composition includes at least one oral care active selected from the group consisting of a teeth whitening active, an anti-tartar agent, a fluoride ion source, an antimicrobial agent, an anti-inflammatory agent, one or more nutrients, a mouth and throat product, an antioxidant, an H2 antagonist, an analgesic active, an anti-viral agent, flavoring agents, sweetening agents, xylitol, opacifiers, coloring agents, chelants, surfactants, pigments, and mixtures thereof.

18. The system of Claim 17 wherein the oral care substance comprises from 0.01 % to 50% of the delivery composition.

19. The system of Claim 18 wherein the oral care substance is a teeth whitening active selected from the group consisting peroxides, metal chlorites, perborates, percarbonates, peroxyacids, persulfates, and mixtures thereof.

20. The use of an organosiloxane resin in the manufacture of a system according to any preceding claim for the delivery of an oral care substance to at least one surface of the oral cavity, wherein the delivery comprises the steps of:
a) applying the delivery composition to the surface(s) of the oral cavity;
b) allowing the composition to form a film on the surface(s) of the oral cavity;
c) applying the protective composition on top of the film formed by the delivery composition; and
d) allowing the protective composition to form a film.

21. The use of Claim 20 wherein the delivery composition comprises a teeth whitening active and the oral cavity surface to which the composition is applied is the enamel of the teeth.

## Patentansprüche

1. System zum Zuführen einer Mundpflegesubstanz zu der Mundhöhle, wobei das System geeignet ist für die Selbstbehandlung und Anwendung zuhause durch einen Anwender und umfasst:
a) eine Zuführzusammensetzung, umfassend
i) ein Organosiloxanharz;
ii) einen flüchtigen Träger, der fähig ist, das Organosiloxanharz zu solubilisieren;
iii) ein Rheologie-Modifiziermittel;
iv) und mindestens eine Mundpflegesubstanz; und
b) eine Schutzzusammensetzung, umfassend:
i) ein Organosiloxanharz; und
ii) einen flüchtigen Träger, der in der Lage ist, das Organosiloxanharz zu solubilisieren.

2. System nach Anspruch 1, wobei die Zuführzusammensetzung weiterhin ein fluides Polymer auf Diorganopolysiloxanbasis umfasst und der flüchtige Träger in der Lage ist, das fluide Polymer auf Diorganopolysiloxanbasis zu solubilisieren.

3. System nach Anspruch 1 und 2, wobei die Schutzzusammensetzung weiterhin ein fluides Polymer auf Diorganopolysiloxanbasis umfasst.

4. System nach Anspruch 2 oder 3, wobei das fluide Polymer auf Diorganopolysiloxanbasis wiederkehrende Einheiten der Formel (R₂SiO)ₙ umfasst, worin R eine einwertige Kohlenwasserstoffrestgruppe mit 1 bis 6 Kohlenstoffatomen ist.

5. System nach Anspruch 4, wobei die Kohlenwasserstoffrestgruppe aus der Gruppe gewählt ist, bestehend aus Methyl, Ethyl, Propyl, Isopropyl, Butyl, Isobutyl, t-Butyl, Amyl, Hexyl, Vinyl, Allyl, Cyclohexyl, Aminoalkyl, Phenyl, Fluoralkyl und Mischungen hiervon.

6. System nach Anspruch 5, wobei das fluide Polymer auf Diorganopolysiloxanbasis terminiert ist durch Triorganosilylgruppen der Formel (R'₃Si), worin R' ein einwertiger Kohlenwasserstoffrest ist, gewählt aus der Gruppe, bestehend aus 1 bis 6 Kohlenstoffatomen, Hydroxylgruppen, Alkoxylgruppen und Mischungen hiervon.

7. System nach Anspruch 6, wobei das fluide Polymer auf Diorganopolysiloxanbasis Poly(dimethylsiloxan) ist.

8. System nach Anspruch 1, wobei das Organosiloxanharz in der Zuführ- und Schutzzusammensetzung in einem Anteil von 5% bis 70% vorliegt.

9. System nach Anspruch 8, wobei das Organosiloxanharz aus der Gruppe gewählt, bestehend aus (CH₃)₃SiO)_{0,5} "M"-Einheiten, (CH₃)₂SiO "D"-Einheiten, (CH₃)SiO_{1,5} "T"-Einheiten, SiO₂ "Q"-Einheiten und Mischungen hiervon.

10. System nach Anspruch 2 oder Anspruch 3, wobei das Verhältnis von Organosiloxanharz zu fluidem Polymer auf Diorganopolysiloxanbasis in der Zuführund Schutzzusammensetzung 15:1 bis 1:15 beträgt.

11. System nach Anspruch 9, wobei das Organosiloxanharz MQ-Einheiten umfasst, wobei das M:Q-Verhältnis 0,5:1,0 bis 1,5:1,0 beträgt.

12. System nach mindestens einem der Ansprüche 1 bis 3, wobei der flüchtige Träger in der Zuführ- und Schutzzusammensetzung aus der Gruppe gewählt ist, bestehend aus Kohlenwasserstoffölen, flüchtigen Siliconen, Nichtkohlenwasserstoff-Lösungsmitteln und Mischungen hiervon.

13. System nach Anspruch 12, wobei der flüchtige Träger aus der Gruppe gewählt ist, bestehend aus Ethanol, Isododecan, Butanon, Ethylacetat, Propylacetat, Methylnonafluorisobutylether, Methylnonafluorbutylether und Mischungen hiervon.

14. System nach mindestens einem der Ansprüche 1 bis 3, wobei die Schutzzusammensetzung weiterhin ein Rheologie-Modifiziermittel umfasst.

15. System nach Anspruch 14, wobei das Rheologie-Modifiziermittel in der Zuführ- und Schutzzusammensetzung aus der Gruppe gewählt ist, bestehend aus organomodifizierten Tonen, Silicas, Polyethylen und Mischungen hiervon.

16. System nach Anspruch 15, wobei das Rheologie-Modifiziermittel in der Zuführ- und Schutzzusammensetzung in einem Anteil von 0,1% bis 30% vorliegt.

17. System nach Anspruch 1 oder Anspruch 2, wobei die Mundpflegesubstanz in der Zuführzusammensetzung mindestens einen Mundpflegewirkstoff umfasst, gewählt aus der Gruppe, bestehend aus einem Zahnbleichwirkstoff, einem Zahnsteinverhinderungsmittel, einer Fluoridionenquelle, einem antimikrobiellen Mittel, einem entzündungshemmenden Mittel, einem oder mehreren Nährstoffen, einem Mund- und Rachenprodukt, einem Antioxidationsmittel, einem H2-Anatagonist, einem analgetischen Wirkstoff, einem antiviralen Mittel, Geschmacksmitteln, Süßungsmitteln, Xylitol, Opazifiziermitteln, Färbemitteln, Komplexbildnern, Tensiden, Pigmenten und Mischungen hiervon.

18. System nach Anspruch 17, wobei die Oralpflegesubstanz 0,01% bis 50% der Zuführzusammensetzung umfasst.

19. System nach Anspruch 18, wobei die Mundpflegesubstanz ein Zahnbleichwirkstoff ist, gewählt aus der Gruppe, bestehend aus Peroxiden, Metallchloriten, Perboraten, Percarbonaten, Peroxysäuren, Persulfaten und Mischungen hiervon.

20. Verwendung eines Organosiloxanharzes bei der Herstellung eines Systems nach mindestens einem vorangehenden Anspruch für die Zufuhr einer Mundpflegesubstanz zu mindestens einer Oberfläche der Mundhöhle, wobei die Zufuhr folgende Schritte umfasst:
a) Aufbringen der Zuführzusammensetzung auf die Oberfläche(n) der Mundhöhle;
b) Bildenlassen eines Films aus der Zusammensetzung auf der(n) Oberfläche(n) der Mundhöhle;
c) Aufbringen der Schutzzusammensetzung auf die Oberfläche des durch die Zuführzusammensetzung gebildeten Films; und
d) Bildenlassen eines Films aus der Schutzzusammensetzung.

21. Verwendung nach Anspruch 20, wobei dei Zuführzusammensetzung einen Zahnbleichwirkstoff umfasst und die Mundhöhlenoberfläche, auf welche die Zusammensetzung aufgebracht wird, der Zahnschmelz der Zähne ist.

## Revendications

1. Système permettant d'administrer une substance d'hygiène buccale à la cavité buccale, le système étant approprié à l'auto-traitement et à l'utilisation à domicile par un utilisateur, et comprenant :
a) une composition d'administration constituée de :
i) une résine organo-siloxane ;
ii) un véhicule volatil capable de solubiliser la résine organo-siloxane ;
iii) un agent modificateur de rhéologie ;
iv) et au moins une substance d'hygiène buccale ; et
b) une composition de protection constituée de :
i) une résine organo-siloxane ; et
ii) un véhicule volatil capable de solubiliser la résine organo-siloxane ;

2. Système selon la revendication 1, dans lequel la composition d'administration comprend en outre un polymère liquide à base de diorgano-polysiloxane, et le véhicule volatil est capable de solubiliser le polymère liquide à base de diorgano-polysiloxane.

3. Système selon les revendications 1 et 2, dans lequel la composition de protection comprend en outre un polymère liquide à base de diorgano-polysiloxane.

4. Système selon la revendication 2 ou 3, dans lequel le polymère liquide à base de diorgano-polysiloxane comprend des motifs récurrents de formule (R₂SiO)ₙ, où R est un groupe radical hydrocarboné monovalent contenant de 1 à 6 atomes de carbone.

5. Système selon la revendication 4, dans lequel ledit groupe radical hydrocarboné est choisi dans le groupe consistant en méthyle, éthyle, propyle, isopropyle, butyle, isobutyle, t-butyle, amyle, hexyle, vinyle, allyle, cyclohexyle, aminoalkyle, phényle, fluoroalkyle et leurs mélanges.

6. Système selon la revendication 5, dans lequel le polymère liquide à base de diorgano-polysiloxane se termine par des groupes triorgano-silyle de formule (R'₃Si), où R' est un radical hydrocarboné monovalent choisi dans le groupe consistant en 1 à 6 atomes de carbone, les groupes hydroxyle, les groupes alcoxyle et leurs mélanges.

7. Système selon la revendication 6, dans lequel le polymère liquide à base de diorgano-polysiloxane est le poly(diméthylsiloxane).

8. Système selon la revendication 1, dans lequel la résine organo-siloxane contenue dans les compositions d'administration et de protection est présente à raison de 5 % à 70 %.

9. Système selon la revendication 8, dans lequel la résine organo-siloxane est choisie dans le groupe consistant en motifs "M" (CH₃)₃SiO_{0,5}, motifs "D" (CH₃)₂SiO, motifs "T" (CH₃)SiO_{1,5}, motifs "Q" SiO₂, et leurs mélanges.

10. Système selon la revendication 2 ou 3, dans lequel le rapport résine organo-siloxane sur polymère liquide à base de diorgano-polysiloxane dans les compositions d'administration et de protection est de 15/1 à 1/15.

11. Système selon la revendication 9, dans lequel la résine organo-siloxane est constituée de motifs MQ, où le rapport M/Q est de 0,5/1,0 à 1,5/1,0.

12. Système selon l'une quelconque des revendications 1 à 3, dans lequel le véhicule volatil présent dans les compositions d'administration et de protection est choisi dans le groupe consistant en huiles d'hydrocarbures, silicones volatils, solvants non hydrocarbonés, et leurs mélanges.

13. Système selon la revendication 12, dans lequel le véhicule volatil est choisi dans le groupe consistant en éthanol, isododécane, butanone, acétate d'éthyle, acétate de propyle, éther méthylnonafluoroisobutylique, éther méthylnonafluorobutylique, et leurs mélanges.

14. Système selon l'une quelconque des revendications 1 à 3, dans lequel la composition de protection comprend en outre un agent modificateur de rhéologie.

15. Système selon la revendication 14, dans lequel l'agent modificateur de rhéologie présent dans les compositions d'administration et de protection est choisi dans le groupe consistant en argiles organo-modifiées, silices, polyéthylène, et leurs mélanges.

16. Système selon la revendication 15, dans lequel l'agent modificateur de rhéologie est présent dans les compositions d'administration et de protection à raison de 0,1 % à 30 %.

17. Système selon la revendication 1 ou 2, dans lequel la substance d'hygiène buccale contenue dans la composition d'administration comprend au moins une substance active d'hygiène buccale choisie dans le groupe consistant en une substance active de blanchiment dentaire, un agent anti-tartre, une source d'ions fluorure, un agent anti-microbien, un agent anti inflammatoire, un nutriment ou davantage, un produit pour la bouche et la gorge, un antioxydant, un antagoniste des récepteurs H2, une substance active analgésique, un agent anti-viral, des aromatisants, des édulcorants, du xylitol, des opacifiants, des colorants, des chélates, des tensioactifs, des pigments, et leurs mélanges.

18. Système selon la revendication 17, dans lequel la substance d'hygiène buccale constitue de 0,01 % à 50 % de la composition d'administration.

19. Système selon la revendication 18, dans lequel la substance d'hygiène buccale est une substance active de blanchiment dentaire choisie dans le groupe consistant en peroxydes, chlorites métalliques, perborates, percarbonates, peroxyacides, persulfates, et leurs mélanges.

20. Utilisation d'une résine organo-siloxane dans la fabrication d'un système selon l'une quelconque des revendications précédentes, permettant d'administrer une substance d'hygiène buccale à au moins une surface de la cavité buccale, l'administration comprenant les étapes consistant à :
a) appliquer la composition d'administration à la(aux) surface(s) de la cavité buccale ;
b) laisser la composition former un film sur la(les) surface(s) de la cavité buccale ;
c) appliquer la composition de protection par dessus le film formé par la composition d'administration ; et
d) laisser la composition de protection former un film.

21. Utilisation de la revendication 20, dans laquelle la composition d'administration comprend une substance active de blanchiment dentaire et dans laquelle la surface de cavité buccale à laquelle est appliquée la composition est l'émail des dents.
